# EUROPEAN PATENT APPLICATION

(11) **EP 2 722 080 A1**
(43) Date of publication of application: **23.04.2014**
(21) Application number: 12189461.2
(22) Date of filing: 22.10.2012
(51) Int. Cl.: A63B 24/00, A61B 5/0488

(54) **Muscle activity training facility**

(71) Applicant: Chen, Paul, Vancouver, BC V7A 1S5 (CA)
(72) Inventor: Chen, Paul, Vancouver, BC V7A 1S5 (CA)
(74) Representative: Beck & Rössig European Patent Attorneys

(57) **Abstract**

A muscle activity training facility includes a monitor (12) electrically connected to a processor (11) and having a screen (15), the screen (15) includes two areas (16, 17), and two figures (18, 19) in one of the areas (16) for indicating the standard operations of the muscle groups in front and rear portions of the user, and two further figures (20, 21) in the other area (17) for indicating the muscle groups in the front and the rear portions of the user, a carrier (30) is attached to the user, and a number of electrodes (32-38) are attached to the carrier (30) and coupled to the processor (11) for detecting the activities of the muscle groups and for showing the detected activities in the other figures (20,. 21) of the screen (15).

## Description

The invention relates to a muscle activity training facility for attaching to limbs of a user and for detecting the muscle activities.

Typical muscle activity training facilities comprise one or more sensors for detecting the muscles of the user. However, the sensors are attached to the hairy portions of the user with tapes or adhesives and may be disengaged from the user after use when the muscles are activated or operated.

The invention is to provide a muscle activity training facility for detecting or training the muscle groups in the limbs of the user.
Figs. 1, 2 are plan views of a muscle activity training facility;
Figs. 3, 4, 5, 6 are partial plan views of a front and a rear portion of a user; and
Figs. 7, 8, 9, 10, 11, 12, 13, 14, 15, 16, 17, 18, 19 are plan views showing the operation of the muscle activity training facility.

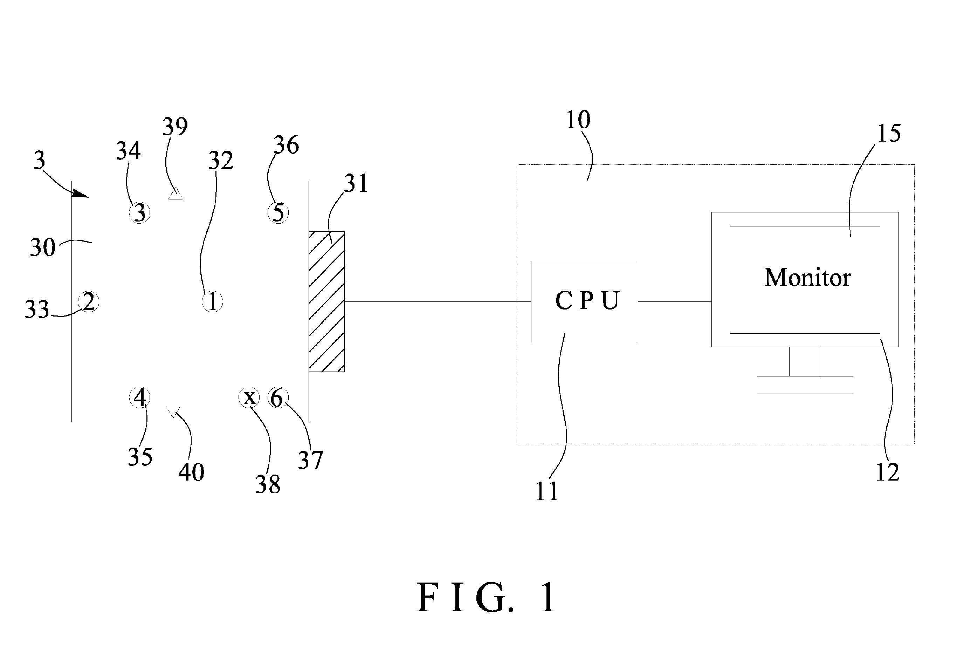

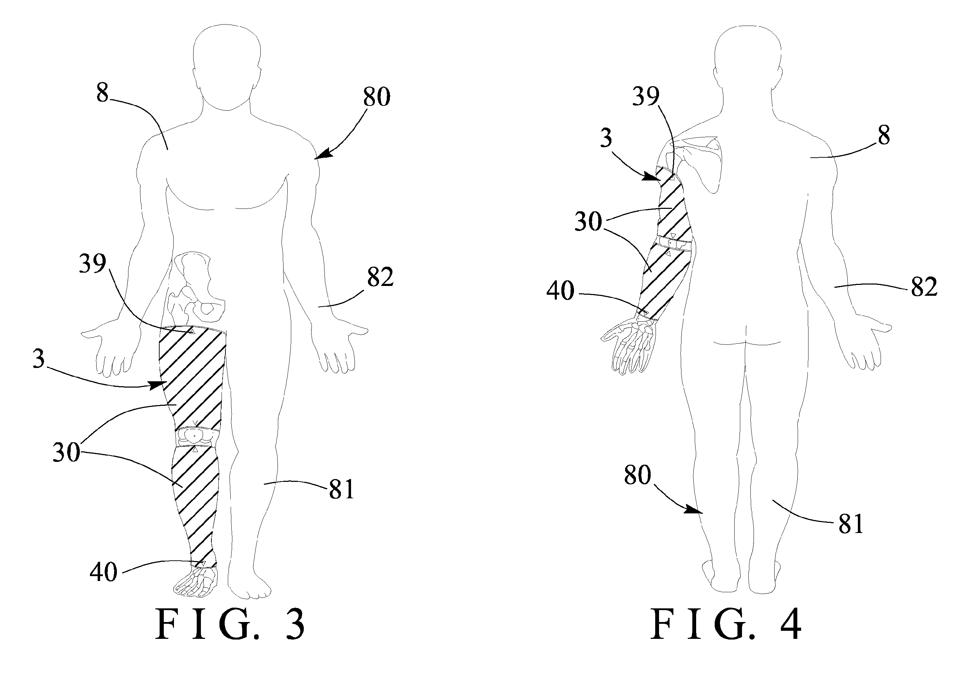

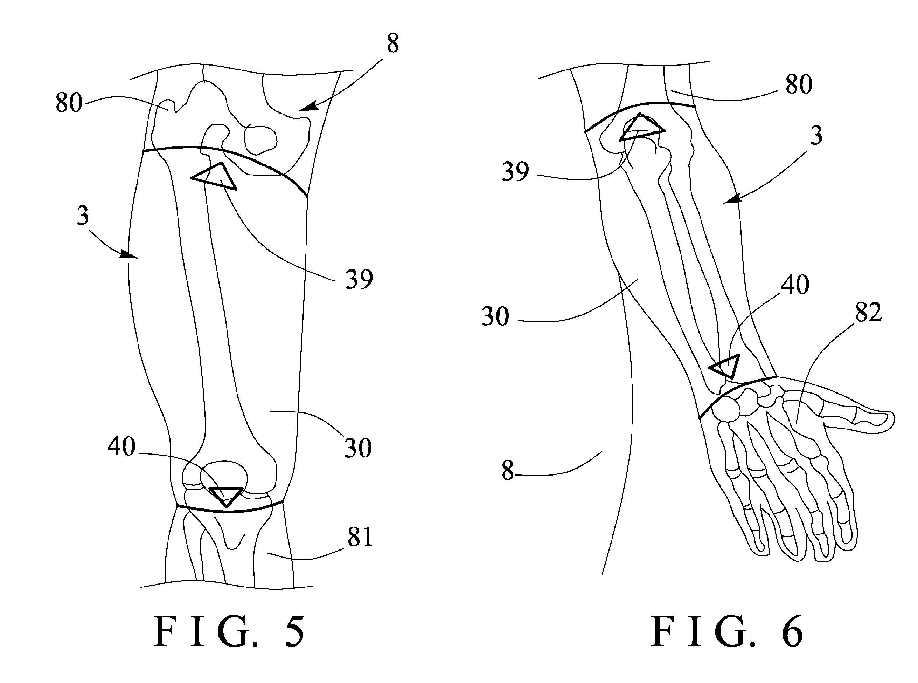

Referring to Fig. 1, a muscle activity training facility comprises a computer 10 including a processor 11 coupled to a monitor 12 which includes a screen 15 for showing information, such as the front and the rear views of the user 8 (Figs. 3-6) or the exercises of the user 8. An adaptor 3 includes a cloth or carrier 30 for attaching to the limbs 80, such as legs 81 (Figs. 3, 5) or hands 82 (Figs. 4, 6) of the user 8, with a Velcro or hook and loop fastening device 31 without being disengaged from the user 8 even when the user 8 is exercising. A number of electrodes 32-37, such as are the electrode 32 is attached to the middle portion of the carrier 30 for engaging onto the inner portion of the limb 80, such as leg 81 or hand 82 (Figs. 3-6), and the other electrodes 33-37 are disposed at the outer and middle portion, the front and upper portion, the front and lower portion, the rear and upper portion, and the rear and lower portion of the limb 80. Another electrode 38, such as a G-sensor 38 is attached to the carrier 30 at the lower portion of the carrier 30 and the terminating portions of the limb 80 for detecting the exercises of the limb 80. The electrodes 32-38 are coupled to send the detected signals to the processor 11.

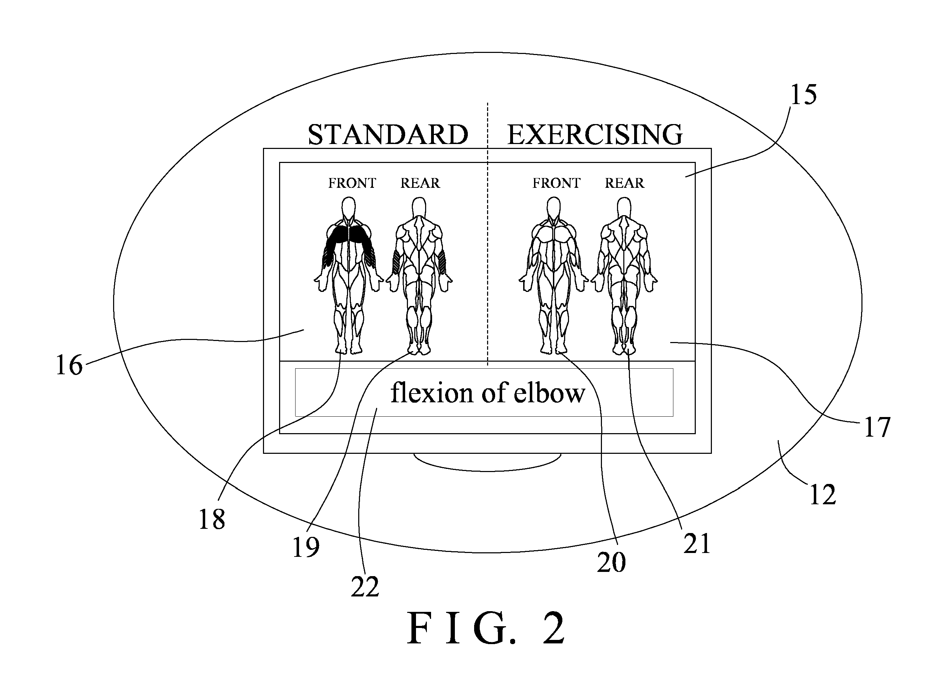

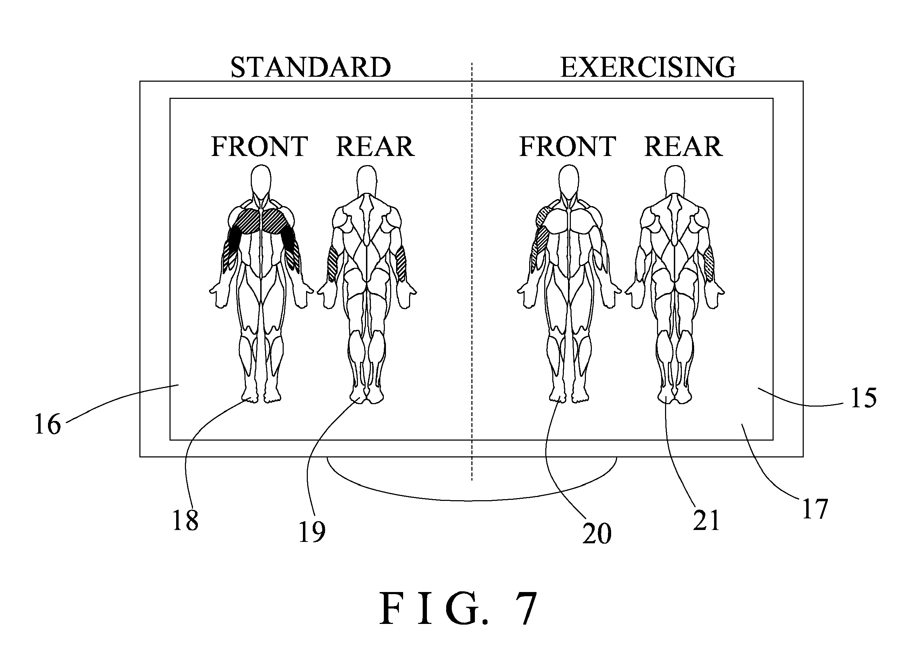

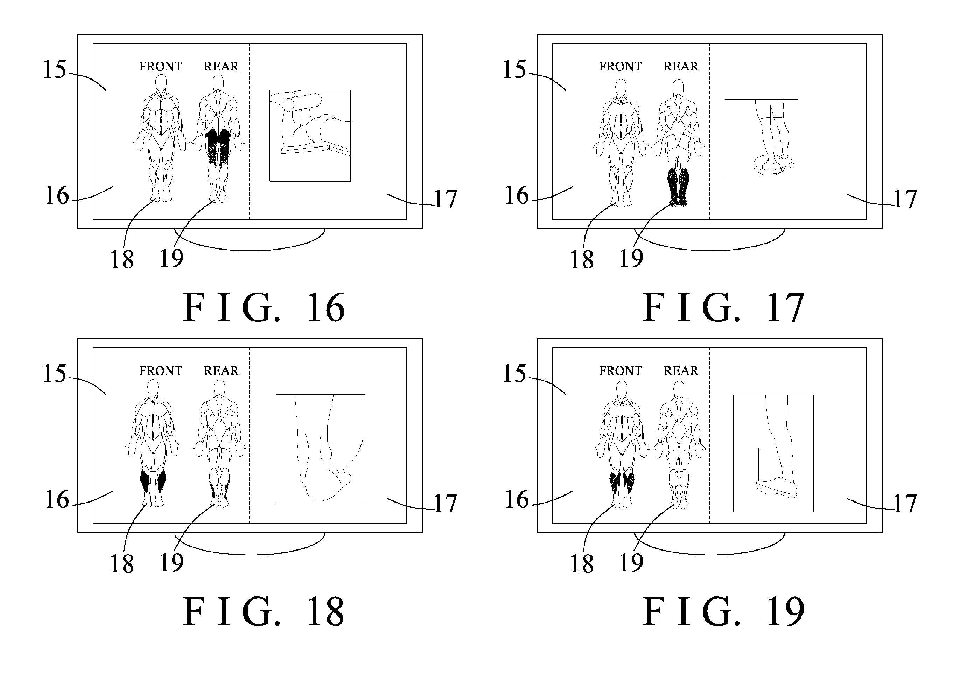

The adaptor 3 includes one or more (such as two) pointers 39, 40 for aligning with selected reference portions of the user 8, such as the upper sternum, the rear acromion process, the rear olecranon process, the rear styloid process, the symphysis pubis, the front portion of the femur head, the patella at the knee joint, or the front portion of the malleolus joint, for aligning the electrodes 32-38 with the selected muscles of the user 8. The screen 15 (Figs.2, 7-19) includes a left or first area 16 having two figures 18, 19 for showing the standard or correct activities of the muscle groups in the front and the rear portions of the user 8 by shaded lines, different colors, the darkness, the brightness or the like (Figs. 7-19), and a right or second area 17 having two figures 20, 21 (Figs. 2, 7) for showing the exercising of the muscle groups in the front and the rear portions of the user 8 and the signals detected by the electrodes 32-38 and/or treated by the processor 11, in which the figures 20,21 may be shown by shaded lines, different colors, the darkness, the brightness or the like for indicating the activities or exercises of the limbs 80 of the user 8.

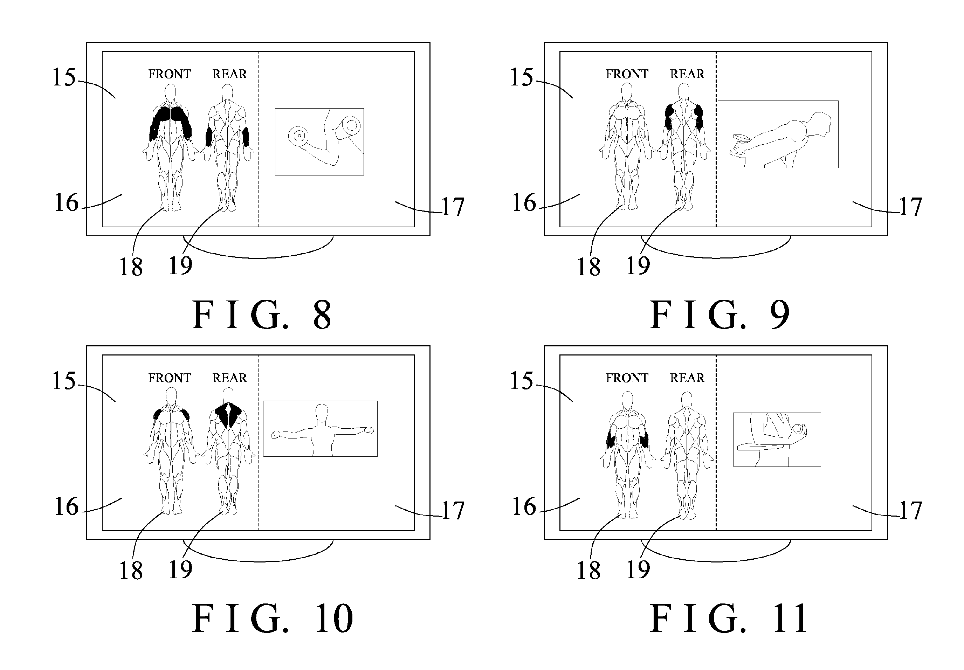

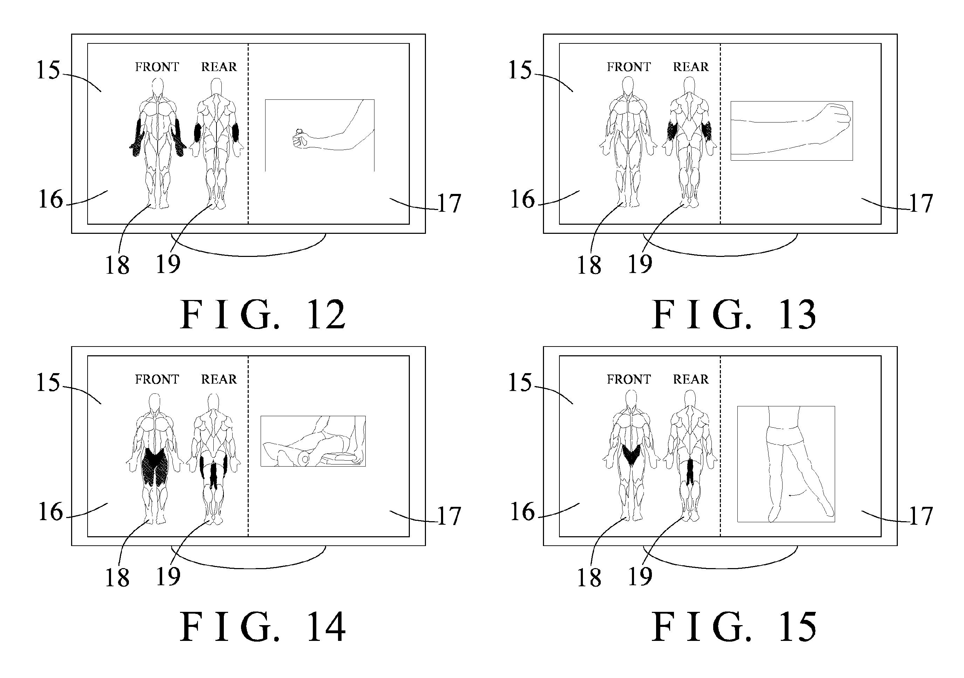

Instead of the figures 20, 21, the second area 17 of the screen 15 (Figs. 8-19) may also be used for displaying the acting of the user 8, such as the biceps brachii muscle is contracted or the flexion of the elbow (Fig. 8), the triceps brachii muscle is extended (Fig. 9), the muscle groups moving the pectoral girdle (Fig. 10), the wrist is curved forwardly (Fig. 11), the lower arm is rotated (Fig. 12), the wrist is curved rearwardly (Fig. 13), the flexion of thigh (Fig. 14), the adduction of thigh (Fig. 15), the flexion of knee (Fig. 16), the flexion of ankle (Fig. 17), the inversion of ankle (Fig. 18), the eversion of ankle (Fig. 19), the proper or correct operations or activities of the muscle groups are shown in the left area 16 of the screen 15 and recorded in the computer 10 as the standard or correct activities of the muscle groups of the user 8. The other operations or activities may also be tested or stored in the computer 10.

In operation, one or more carriers 30 may be attached to the user 8 (Figs. 3-6) with the fastening device 31 by aligning the pointers 39, 40 with the selected reference portions of the user 8, the user may then select the required activity to be conducted (Fig. 2), such as the flexion of the elbow, which will be shown in the lower or selected area 22 of the screen 15, and/or shown in the second area 17 of the screen 15 (Figs. 8-19). When exercising (Figs. 2, 7), the figures 20, 21 in the second area 17 of the screen 15 may indicate the activities of the muscle groups in the limbs 80 of the user 8. The user 8 may then compare the figures 20, 21 with the figures 18, 19 at the first area 16 of the screen 15, and may then determine the difference between the muscle groups in the figures 18-21, in order to correct the activity or exercise operation of the user 8.

## Claims

1. A muscle activity training facility comprising a processor (11), a monitor (12) connected to the processor (11) and having a screen (15), **characterized in that:**
the screen (15) includes a first area (16) and a second area (17), two first figures (18, 19) in the first area (16) for standard operations, two second figures (20, 21) in the second area (17) for indicating operations of muscle groups in the front and the rear portions of a user, a carrier (30) for attaching to the user, and a plurality of electrodes (32-37) attached to the carrier (30) and coupled to the processor (11).

2. A muscle activity training facility as claimed in claim 1, wherein the carrier (30) includes a fastening device (31) for securing the carrier (30).

3. A muscle activity training facility as claimed in claim 2, wherein the fastening device (31) is a hook and loop fastening device (31).

4. A muscle activity training facility as claimed in one of claims 1 to 3, wherein the carrier (30) includes a G-sensor (38).

5. A muscle activity training facility as claimed in one of claims 1 to 4, wherein the monitor (12) includes a selected area (22) of the screen (15).

6. A muscle activity training facility as claimed in one of claims 1 to 5, wherein the carrier (30) includes at least one pointer (39, 40).

7. A muscle activity training facility as claimed in one of claim 1 to 6, wherein the carrier (30) is a flexible planar member.
